(19) Europäisches Patentamt
European Patent Office
Office européen des brevets

(11) **EP 1 722 841 B1**

(12) **EUROPÄISCHE PATENTSCHRIFT**

(45) Veröffentlichungstag und Bekanntmachung des Hinweises auf die Patenterteilung:
**10.03.2021 Patentblatt 2021/10**

(21) Anmeldenummer: **04804323.6**

(22) Anmeldetag: **27.12.2004**

(51) Int Cl.:
***A61M 5/172*** *(2006.01)*

(86) Internationale Anmeldenummer:
**PCT/EP2004/014733**

(87) Internationale Veröffentlichungsnummer:
**WO 2005/084733 (15.09.2005 Gazette 2005/37)**

(54) **VORRICHTUNG ZUM BERECHNEN EINER BOLUSMENGE**

DEVICE FOR CALCULATING A BOLUS AMOUNT

DISPOSITIF POUR CALCULER UNE QUANTITE DE BOLUS

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **08.03.2004 DE 102004011135**

(43) Veröffentlichungstag der Anmeldung:
**22.11.2006 Patentblatt 2006/47**

(73) Patentinhaber:
• **F. Hoffmann-La Roche AG**
**4070 Basel (CH)**
Benannte Vertragsstaaten:
**AT BE BG CH CY CZ DK EE ES FI FR GB GR HU IE IS IT LI LT LU MC NL PL PT RO SE SI SK TR**
• **Roche Diabetes Care GmbH**
**68305 Mannheim (DE)**
Benannte Vertragsstaaten:
**DE**

(72) Erfinder: **HELLWIG, Robert**
**CH-3012 Bern (CH)**

(74) Vertreter: **Rentsch Partner AG**
**Bellerivestrasse 203**
**Postfach**
**8034 Zürich (CH)**

(56) Entgegenhaltungen:
**EP-A2- 1 281 351        DE-A1- 3 545 260**
**DE-A1- 10 057 215       US-A1- 2003 055 570**
**US-A1- 2003 104 982     US-B1- 6 554 798**

EP 1 722 841 B1

## Beschreibung

Die vorliegende Erfindung bezieht sich auf eine Vorrichtung zum Berechnen einer Bolusmenge gemäß dem Oberbegriff des Anspruchs 1.

Ein Mensch sollte etwa zwischen 70 und 126 mg Glukose pro dl Blut haben, wobei die im Blut vorhandene Glukose mit Hilfe von Insulin in Körperzellen verbrannt werden kann, um die für den Organismus lebenswichtige Energie zur Verfügung zu stellen. Gewöhnlich wird das benötigte Insulin von der Bauchspeicheldrüse erzeugt, welche die erforderliche Menge des Insulins produziert und an den Organismus abgibt. Diabetiker benötigen jedoch eine künstliche Zufuhr von Insulin, wobei häufig Insulinpumpen verwendet werden, welche die Funktion der nicht mehr richtig funktionierenden Bauchspeicheldrüse vollständig oder zumindest zum Teil übernehmen sollen. Dabei werden kontinuierlich kleinere Mengen des Insulins über den Tag verteilt mittels eines Katheters meist abdominal in das Unterhautfettgewebe eines Patienten infundiert, wobei eine einem Patienten über den Tag verteilt kontinuierlich zugeführte und von der Tageszeit abhängige Menge des Insulins auch als Basalrate bezeichnet wird. Der Bedarf an Basal-Insulin ist von Patient zu Patient verschieden und unterliegt tagesrhythmischen Schwankungen, so dass das Basalratenprofil in eine Insulinpumpe patientenspezifisch einprogrammiert wird. Wenn Nahrung aufgenommen wird, wird eine größere Menge an Insulin, ein sogenannter Bolus, benötigt, um die über die Nahrung aufgenommene Menge an Kohlenhydraten bzw. Zucker für die Körperzellen verwertbar zu machen.

Somit muss ein Patient jedes Mal dann, wenn er Kohlenhydrate essen will oder einen erhöhten Blutzucker korrigieren will, die Abgabe von Bolus-Insulin veranlassen. Hierzu sollte die erforderliche Bolusmenge möglichst genau berechnet werden. Eine Möglichkeit eine Empfehlung für die abzugebende Menge Insulin zu errechnen ist die Verwendung der folgenden Formel 1:

$$
\begin{aligned}
\text{Rec [I.U.]} = \ & x_{carbohydrates}\ [g] * A_{meal}\ [I.U. *g^{-1}] \\
+ \ & (x_{BGactual}\ [mmol*l^{-1}] - B_{BGtarget}[mmol*l^{-1}] - C_{insulin\ still\ effective}[I.U.]*D_{correction} \\
& [mmol*l^{-1}*I.U.^{-1}]) \\
& *(D_{correction}\ [mmol*l^{-1}*I.U.^{-1}])^{-1}
\end{aligned}
$$

In der oben bezeichneten Formel 1 bezeichnet die Variable Rec [I.U.] die durch die Formel berechnete Empfehlung der als Bolus zu verabreichenden Insulineinheiten I.U.. Zur Berechnung der Empfehlung der abzugebenden Insulineinheiten werden zwei variable Parameter verwendet, nämlich der Parameter $x_{carbohydrates}$, welcher die Menge der als Nahrung aufzunehmenden oder aufgenommenen Kohlenhydrate in Gramm angibt und der Parameter $x_{BG}$ actual, welcher den gemessenen oder tatsächlichen Blutzuckerwert in mmol/l angibt. Weiterhin werden als vorgegebene Parameter verwendet: $A_{meal}$, welcher die Menge der Insulineinheiten angibt, um ein Gramm Kohlenhydrate zu verarbeiten bzw. zu kompensieren, $B_{BG\ target}$, welcher den Ziel- oder Sollwert des Blutglukosewertes in mmol/l angibt und $D_{correction}$, welcher in mmol/l pro einer Einheit des Insulins angibt, wie der Blutglukosewert verringert wird durch den Einfluss einer Einheit Insulin. Der Parameter $C_{Insulin\ still\ effective}$ wird verwendet, um bereits injiziertes oder infundiertes Insulin zu berücksichtigen, wobei dieser Parameter basierend auf Algorithmen berechnet werden kann, welche die noch vorhandene Aktivität des infundierten Insulins im Körper zu einem gegebenen Zeitpunkt nach der Infusion berechnen können. Es kann z.B. die verabreichte Menge an Insulin über einen vergangenen Zeitraum von zum Beispiel 6 bis 12 Stunden berücksichtigt werden.

Wird die oben dargestellte Formel 1 zur Berechnung einer Bolus-Dosierung verwendet, werden für die Parameter $A_{meal}$ und $D_{correction}$ in Abhängigkeit von der Tageszeit über vorgegebene Zeiträume konstante Werte vorgegeben. Zum Beispiel wird für den Parameter $A_{meal}$ für die Zeit zwischen 22:00 Uhr und 06:00 Uhr ein Wert von 0,9 Insulineinheiten (I.U.) pro Broteinheit (entsprechend 12g Kohlenhydrate) festgelegt. Für den Zeitraum zwischen 6:00 Uhr morgens und 10:00 Uhr morgens wird ein Wert von 1,5 Insulineinheiten pro Broteinheit, zwischen 10:00 Uhr und 16:00 Uhr ein Wert von 1,0 Insulineinheiten pro Broteinheit und zwischen 16:00 Uhr und 22:00 Uhr ein Wert von 1,4 Insulineinheiten pro Broteinheit festgelegt. Entsprechend wird dem Parameter $D_{correction}$ ein konstanter Wert in Abhängigkeit von der Tageszeit zugewiesen und basierend auf diesen den Parametern $A_{meal}$ und $D_{correction}$ zugewiesenen Konstanten wird unter Verwendung der oben genannten Formel 1 eine Empfehlung der als Bolus abzugebenden Insulineinheiten berechnet.

Aus der US 6,691,043 B2 ist ein Verfahren zur Berechnung eines Boluswertes basierend auf einer täglich variierenden Voraussage der benötigten Insulinmenge bekannt DE 10057215A1 zeigt ein System zur Extrapolation einer Glucosekonzentration sowie zur Ermittlung zu verabreichender Insulinangaben.

US 2003/0055570 betrifft das Ermitteln einer Menge Insulin für eine Mahlzeit aufgrund einer Glukosemessung, einer Menge gegessener Kohlenhydrate und einer Vorhersage für ein Menge Insulin für den betreffenden Tag.

Es ist eine Aufgabe der Erfindung, eine Vorrichtung vorzuschlagen, mit welchen einfach und zuverlässig eine Bolusempfehlung ermittelt werden kann.

Diese Aufgabe wird durch die Vorrichtung gemäß dem unabhängigen Anspruch 1 gelöst. Vorteilhafte Ausführungsformen ergeben sich aus den Unteransprüchen.

Bei der erfindungsgemäßen Vorrichtung wird zum Berechnen einer Bolusmenge bzw. einer Empfehlung für die abzugebenden Insulineinheiten zur Kompensation einer von einem Benutzer aufgenommenen Menge Kohlenhydrate die momentane Basalrate verwendet, das heißt, die Bolusmenge wird in Abhängigkeit von der aktuellen

Basalrate bzw. als Funktion der Basalrate oder des Basalratenprofils berechnet. Der erfindungsgemäßen Vorrichtung liegt die Erkenntnis zu Grunde, dass die sich über den Tagesverlauf stetig verändernde Basalrate mit den oben beschriebenen Faktoren $A_{meal}$ und $D_{correction}$ korreliert ist. Somit werden bei der erfindungsgemäßen Vorrichtung zum Berechnen einer Bolusmenge bzw. eines Vorschlags zur Abgabe einer Insulindosis als Bolus keine Parameter verwendet, welche sich nach vorgegebenen Zeiträumen sprungartig verändern, sondern es wird über den Tag verteilt zu jedem Zeitpunkt eine Bolusmenge berechnet, welche dem Tagesrhythmus entsprechend möglichst optimal an die individuelle Situation eines Patienten angepasst ist.

Vorzugsweise wird der oben in Formel 1 enthaltene Parameter $A_{meal}$ durch nachfolgende Formel 2 berechnet:

$$A'_{meal} \; [I.U. * g^{-1}] = CF_A \; [h*g^{-1}] * BR_{actual} \; [I.U. * h^{-1}]$$

Ebenso wird bevorzugt der in Formel 1 enthaltene Faktor $D_{correction}$ durch nachfolgende Formel 3 berechnet:

$$D'_{correction} \; [mmol * l^{-1} * I.U.^{-1}] = CF_D \; [mmol * l^{-1} * h * I.U.^{-2}] * BR_{actual} \; [I.U. * h^{-1}]$$

Durch die obigen Formeln 2 und 3 können die Parameter $A'_{meal}$ und $D'_{correction}$ als eine Funktion der momentanen Basalrate berechnet werden, wobei die momentane Basalrate $BR_{actual}$ mit den konstanten Faktoren $CF_A$ bzw. $CF_D$ multipliziert wird. Der Faktor $CF_A$ ist eine Konstante, welche die Korrelation zwischen dem Parameter $A_{meal}$ und der momentanen Basalrate $BR_{actual}$ beschreibt, welche zum Beispiel die momentane Basalrate in Insulineinheiten pro Stunde angibt. Entsprechend ist der Faktor $CF_D$ eine Konstante, welche die Korrelation zwischen dem Parameter $D_{correction}$ und der momentanen Basalrate $BR_{actual}$ beschreibt.

Die aktuelle oder momentane Basalrate $BR_{actual}$ kann z.B. in einem Speicher eines PCs, eines Organizers, einer Pumpe oder eines anderen bevorzugt tragbaren Gerätes abgelegt sein oder kann aus z.B. aus einer Menge des Insulintagesbedarfs ermittelt werden. Hierzu kann z.B. ein bekanntes Verfahren nach Renner et al. verwendet werden, wie es z.B. in der Broschüre "Informationen zur Insulinpumpen-Therapie (CSII)" von Disetronic, welche nach Inhalten der Insulinpumpen-Seminare des Diabeteszentrums am Klinikum München-Bogenhausen zusammengestellt worden ist, beschrieben ist. Ergänzend wird auf den Artikel "PROSPEKTIVE EVALUATION EINER STANDARDISIERTEN BASALRATENVERTEILUNG DIE CSII BEI TYP-I-DIABETES ÜBER 6 MONATE" von Wizemann, E., Renner, R., Hepp, K. D. aus der III. Med. Abteilung und Diabeteszentrum Krankenhaus München Bogenhausen, veröffentlicht als Abstract in DIABETES UND STOFFWECHSEL, ZEITSCHRIFT FÜR ANGEWANDTE DIA-BETOLOGIE, 36. Jahrestagung der DEUTSCHEN DIABETES-GESELLSCHAFT vom 23.-26. Mai 2001 in Aachen, Band 10, Supplement-Heft 1, Mai 2001 verwiesen.

Werden in obiger Formel 1 die Parameter $A_{meal}$ und $D_{correction}$ durch die in den Formeln 2 und 3 definierten Parameter $A'_{meal}$ und $D'correction$ ersetzt, wird die berechnete Empfehlung der Bolusmenge Rec eine Funktion der momentanen sich stetig mit der Zeit verändernden Basalrate $BR_{actual}$ und weist somit keine Sprünge auf wie im Fall der Verwendung von Schätzwerten für $A_{meal}$ und $D_{correction}$, welchen für vorgegebene Tageszeiten konstante Werte zugewiesen werden.

Der Faktor $CF_A$ in Formel 2 kann berechnet werden, wenn mindestens ein Wert für $A_{meal}$ zusammen mit der Information bezüglich der Zeit, zu welcher dieser Parameter $A_{meal}$ gültig ist, bekannt ist. Allgemein kann der Faktor $CF_A$ durch nachfolgend dargestellte Formel 4 berechnet werden:

$$(CF_A)_i \; [h * g^{-1}] = n * \left( \sum_{k=1}^{n} BR_k \; [I.U. * h^{-1}] \right)^{-1} * (A_{meal})_i [I.U. * g^{-1}]$$

Dabei ist $k \geq 1$ und ein Index für die bestimmten Stunden des Tages, bei welchen $A_{meal}$ gültig ist. $n \geq 1$ bezeichnet die Anzahl der Stunden, zu welchen $A_{meal}$ gültig ist. $i \geq 1$ ist ein Index für die einzelnen Faktoren $A_{meal}$ und $CF_A$. $(A_{meal})_i$

bezeichnet den Faktor $A_{meal}$, welcher während der Stunden k gültig ist. $(CF_A)_i$ ist ein konstanter Faktor $CF_A$, welcher aus $(A_{meal})_i$ erhalten wird.

Wenn nur ein Faktor $A_{meal}$ verwendet wird, um $CF_A$ zu bestimmen, gilt folgende Formel 5:

$$CF_A[h*g^{-1}]=(CF_A)_i[h*g^{-1}]$$

Wenn mehr als nur ein Faktor $A_{meal}$ verwendet wird, um $CF_A$ zu bestimmen, gilt folgende Formel 6:

$$CF_A[h*g^{-1}] = n^{-1}\sum_{i=1}^{n}(CF_A)_i[h*g^{-1}]$$

Entsprechend wird der Faktor $CF_D$ allgemein durch die folgende Formel 7 berechnet:

$$(CF_D)_i[mmol*l^{-1}*h*I.U.^{-2}]=n*\left(\sum_{k=1}^{n}BR_k[I.U.*h^{-1}]\right)^{-1}*(D_{correction})_i[mmol*l^{-1}*I.U.^{-1}]$$

Dabei ist $k \geq 1$ und ein Index für die bestimmten Stunden des Tages, bei welchen $D_{correction}$ gültig ist. $n \geq 1$ bezeichnet die Anzahl der Stunden, zu welchen $D_{correction}$ gültig ist. $i \geq 1$ ist ein Index für die einzelnen Faktoren $D_{correction}$ und $CF_D$. $(D_{correcti-on})_i$ bezeichnet den Faktor $D_{correction}$, welcher während der Stunden k gültig ist. $(CF_D)_i$ ist ein konstanter Faktor $CF_D$, welcher aus $(D_{correction})_i$ erhalten wird.

Wenn nur ein Faktor $D_{correction}$ verwendet wird, um $CF_D$ zu bestimmen, gilt die folgende Formel 8:

$$CF_D[mmol*l^{-1}*h*I.U.^{-2}]=(CF_d)_i[mmol*l^{-1}*h*I.U.^{-1}]$$

Wenn mehr als nur ein Faktor $D_{correction}$ verwendet wird, um $CF_D$ zu bestimmen, gilt die folgende Formel 9:

$$(CF_D)[mmol*l^{-1}*h*I.U.^{-2}] = n^{-1}\sum_{i=1}^{n}(CF_D)_i[mmol*l^{-1}*h*I.U.^{-2}]$$

Somit kann durch die erfindungsgemäße Vorrichtung durch die direkte Einbeziehung der Basalrate über oben beschriebene Formeln eine Bolusmenge berechnet werden, welche individuell besser an einen Patienten angepasst ist und keine Sprungstellen über die Zeit aufweist, da die Bolusmenge basierend auf der sich mit der Zeit stetig verändernden Basalrate ermittelt wird.

Die Vorrichtung zum Bestimmen oder Berechnen einer Menge an Insulin, welche einer Person als Bolus verabreicht werden soll, um einen geeigneten Blutzuckerwert aufrechtzuerhalten, ist mit einer Eingabeeinheit, wie zum Beispiel einer Tastatur oder einem Touch-Screen, zum Eingeben einer Menge an Kohlenhydraten $x_{carbohydrates}$, welche eine Person aufnehmen will und zum Eingeben des momentanen Blutglukosewertes $X_{BG\ actual}$ versehen, wobei diese Parameter zum Beispiel auch über eine Schnittstelle an die erfindungsgemäße Vorrichtung übermittelt werden können. Weiterhin weist die Vorrichtung eine Recheneinheit auf, welche basierend auf den eingegebenen Werten für $X_{carbohydrates}$ und $x_{BG\ actual}$ eine Empfehlung Rec für die Bolusmenge nach dem oben beschriebenen Verfahren berechnet. Es ist eine Ausgabeeinheit vorgesehen, über welche die von der Recheneinheit ermittelte Empfehlung Rec ausgegeben wird und zum Beispiel auf einem Display dargestellt und/oder über eine Schnittstelle an eine Infusionspumpe übermittelt wird.

Die erfindungsgemäße Vorrichtung kann eine bekannte Blutglukosemessvorrichtung aufweisen, mit welcher der momentane Blutglukosewert $x_{BG\ actual}$ gemessen und an die erfindungsgemäße Vorrichtung übertragen wird, so dass ein Benutzer zum Berechnen einer erforderlichen Bolusmenge nur noch die Menge der beabsichtigten Aufnahme an Kohlenhydraten $X_{carbohydrates}$ in die erfindungsgemäß Vorrichtung eingeben muss.

Bevorzugt ist an der erfindungsgemäßen Vorrichtung ein Speicher, wie zum Beispiel ein RAM vorgesehen, um zum Beispiel Werte für die sich über den Tag verändernde Basalrate BR abzuspeichern und/oder um zu speichern wie viel Insulin zu welchem Zeitpunkt bereits abgegeben wurde.

Vorzugsweise ist ein Zeitmesser, wie zum Beispiel eine Uhr vorgesehen, um in Abhängigkeit von der Tageszeit einen geeigneten Wert einer Bolusmenge ermitteln zu können und um zum Beispiel mit einer Zeitstempelfunktion eine abgegebene Menge des Insulins in einem Speicher für weitere Berechnungen abzulegen.

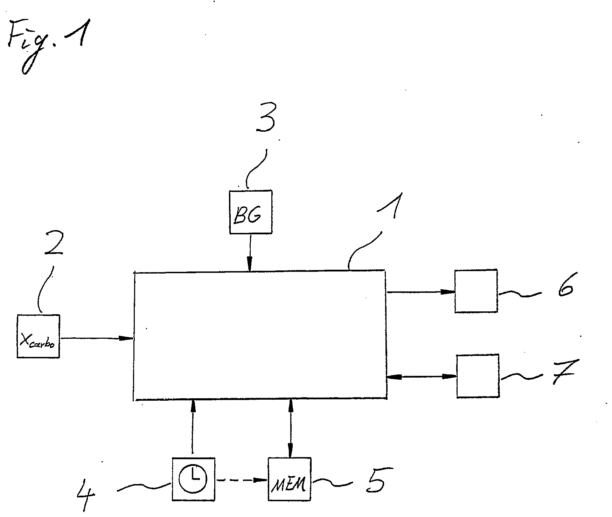

Die Erfindung wird nachfolgend anhand eines in Figur 1 gezeigten Ausführungsbeispieles beschrieben. Dabei zeigt:

Figur 1    eine Ausführungsform der erfindungsgemäßen Vorrichtung zum Berechnen einer Bolusmenge.

Figur 1 zeigt eine Ausführungsform der erfindungsgemäßen Vorrichtung zum Berechnen einer Bolusmenge mit einer Recheneinheit 1, welche mit einer Eingabeeinheit 2, wie zum Beispiel einer Tastatur, zum Eingeben der Menge der Kohlenhydrate $x_{carbohydrates}$, welche von einem Anwender aufgenommen wird oder aufgenommen worden ist, gekoppelt ist. Weiterhin ist die Recheneinheit 1 mit einem Sensor 3 zur Messung des momentanen Blutzuckerwertes gekoppelt. Ein Zeitgeber, wie zum Beispiel eine Uhr 4, gibt die Information bezüglich der Tageszeit an die Recheneinheit 1 und optional an einen Speicher 5 weiter, welcher von der Recheneinheit 1 ausgelesen und beschrieben werden kann, um zum Beispiel die Menge des abgegebenen Insulins zusammen mit dem Zeitpunkt der Abgabe im Speicher 5 abzuspeichern und für die neue Berechnung einer Bolusmenge auszulesen. Die von der Recheneinheit 1 ermittelte Bolusmenge kann an eine Anzeige, wie zum Beispiel einen Bildschirm oder ein LCD-Display 6, ausgegeben werden. Ebenso kann die von der Recheneinheit 1 ermittelte Bolusmenge direkt an eine Insulinpumpe 7 ausgegeben werden, welche unmittelbar die Abgabe von schnellwirkendem Insulin entsprechend der ermittelten Bolusmenge veranlasst.

In der Pumpe 7 kann, ebenso wie in dem Speicher, 5 der zeitliche Verlauf der Basalrate gespeichert sein, welcher der Recheneinheit 1 von der Pumpe 7 und/oder dem Speicher 5 zur erfindungsgemäßen Berechnung einer Bolusmenge zur Verfügung gestellt wird.

Im Speicher 5 können die oben beschriebenen weiteren Faktoren bzw. Parameter zur Berechnung der Bolusmenge abgelegt sein, welche zum Beispiel über die Eingabeeinheit 2 beim Konfigurieren der erfindungsgemäßen Vorrichtung eingegeben worden sind.

**Patentansprüche**

1.  Vorrichtung zum Berechnen und Ausgeben einer Bolusmenge (Rec) mit

    a) einer Eingabeeinheit (2) zum Eingeben einer Menge von aufgenommenen Kohlehydraten ($X_{carbohydrates}$) einer Person,
    b) einer Recheneinheit (1), welche mit der Eingabeeinheit (2) gekoppelt ist, wobei die eingegebene Menge von aufgenommenen Kohlehydraten ($X_{car-bohydrates}$) an die Recheneinheit (1) übertragbar ist, wobei die Recheneinheit

    - mindestens eine Schnittstelle, über welche der momentane Blutglucosewert ($X_{BG\ actual}$) der Person an die Recheneinheit (1) übertragbar ist und
    - eine Ausgabeeinheit aufweist zur Ausgabe einer von der Recheneinheit (1) berechneten Bolusmenge (Rec),

    **dadurch gekennzeichnet, dass**
    über mindestens eine Schnittstelle der Recheneinheit (1) der Wert einer momentanen Basalrate ($BR_{actual}$) der Person an die Recheneinheit (1) übertragbar ist und dass die Recheneinheit (1) ausgestaltet ist, um die Bolusmenge (Rec) in Abhängigkeit von der Menge von aufgenommenen Kohlehydraten ($X_{carbohydrates}$) und in Abhängigkeit von der momentanen Basalrate ($BR_{actual}$) zu berechnen und die berechnete Bolusmenge (Rec) auf der Ausgabeeinheit auszugeben.

2.  Vorrichtung nach Anspruch 1 mit einer Messvorrichtung (3) zum Messen des aktuellen Blutglucosewerts ($X_{BG\ actual}$) der Person unter Übertragung über die mindestens eine Schnittstelle der Recheneinheit (1) des aktuellen Blutglucosewerts ($X_{BG\ actual}$) an die Recheneinheit (1), wobei die Recheneinheit ausgestaltet ist, die Bolusmenge (Rec) in Abhängigkeit des aktuellen Blutglucosewerts ($X_{BG\ actual}$) zu berechnen.

3.  Vorrichtung nach Anspruch 1 oder 2 mit einem Zeitmesser (4), welcher mit der Recheneinheit (1) gekoppelt ist, um die Tageszeit an die Recheneinheit (1) zu übertragen.

4.  Vorrichtung nach einem der Ansprüche 1 bis 3 mit einem Speicher (5), welcher mit der Recheneinheit (1) und/oder einem Zeitmesser (4) gekoppelt ist, um für die Berechnung der Bolusmenge vorgegebene Parameter und/oder die Menge des abgegebenen Insulins und/oder um den zeitlichen Verlauf der Basalrate (BR) zu speichern.

**5.** Vorrichtung nach einem der Ansprüche 1 bis 4 mit einer Anzeigevorrichtung (6), welche mit der Ausgabeeinheit verbunden ist, um die von der Recheneinheit (1) berechnete Bolusmenge (Rec) anzuzeigen.

**6.** Vorrichtung nach einem der Ansprüche 1 bis 5 mit einer Insulinpumpe (7), welche mit der Recheneinheit (1) gekoppelt ist, um Informationen bezüglich der Bolusmenge (Rec) und/oder der momentanen Basalrate ($BR_{actual}$) auszutauschen.

**Claims**

**1.** A device for calculating and outputting a bolus amount (Rec) comprising

a) an input unit (2) for inputting an amount of consumed carbohydrates ($X_{carbohydrates}$) by a person,
b) a computing unit (1) which is coupled to the input unit (2), wherein the input amount of consumed carbohydrates ($X_{carbohydrates}$) is transmittable to the computing unit (1), the computing unit having

- at least one interface, via which the current blood glucose value ($X_{BG\ actual}$) of the person is transmittable to the computing unit (1), and
- an output unit for outputting a bolus amount (Rec) calculated by the computing unit (1),

**characterised in that**
the value of a current basal rate ($BR_{actual}$) of the person is transmittable to the computing unit (1) via at least one interface of the computing unit (1), and **in that** the computing unit (1) is designed to calculate the bolus amount (Rec) depending on the amount of consumed carbohydrates ($X_{carbohydrates}$) and depending on the current basal rate ($BR_{actual}$) and to output the calculated bolus amount (Rec) on the output unit.

**2.** Device according to claim 1 comprising a measuring device (3) for measuring the current blood glucose value ($X_{BG\ actual}$) of the person with transmission via the at least one interface of the computing unit (1) of the current blood glucose value ($X_{BG\ actual}$) to the computing unit (1), wherein the computing unit is designed to calculate the bolus amount (Rec) in dependence of the current blood glucose value ($X_{BG\ actual}$) .

**3.** Device according to claim 1 or 2 with a timing unit (4) which is coupled to the computing unit (1) in order to transmit the time of day to the computing unit (1).

**4.** Device according to one of claims 1 to 3 with a memory (5) which is coupled to the computing unit (1) and/or a timing unit (4) in order to store pre-set parameters for the calculation of the bolus amount and/or the amount of delivered insulin and/or the temporal course of the basal rate (BR).

**5.** Device according to one of claims 1 to 4 with a display device (6) which is connected to the output unit in order to display the bolus amount (Rec) calculated by the computing unit (1).

**6.** Device according to one of claims 1 to 5 comprising an insulin pump (7) which is coupled to the computing unit (1) in order to exchange information regarding the bolus amount (Rec) and/or the current basal rate ($BR_{actual}$) .

**Revendications**

**1.** Dispositif de calcul et de délivrance d'une quantité de bolus (Rec), comportant

a) une unité d'incorporation (2) d'une quantité de glucides absorbés ($X_{glucides}$) d'une personne,
b) une unité de calcul (1) qui est couplée à l'unité d'incorporation (2), la quantité incorporée de glucides absorbés ($X_{glucides}$) étant transmissible à l'unité de calcul (1), l'unité de calcul présentant

- au moins une interface par laquelle le taux momentané de glucose dans le sang ($X_{BG\ actuel}$) de la personne est transmissible à l'unité de calcul (1) et
- une unité de délivrance pour délivrer une quantité de bolus calculée (Rec) par l'unité de calcul (1),

**caractérisé en ce que**,

par au moins une interface de l'unité de calcul (1), la valeur d'un taux basal momentané (BR$_{actuel}$) de la personne est transmissible à l'unité de calcul (1) et que l'unité de calcul (1) est conçue pour délivrer la quantité de bolus (Rec) en fonction de la quantité de glucides absorbés (X$_{glucides}$) en fonction du taux basal momentané (BR$_{actuel}$) et de la quantité de bolus calculée (Rec) sur l'unité de délivrance.

2. Dispositif selon la revendication 1, comportant un dispositif de mesure (3) du taux actuel de glucose dans le sang (X$_{BG}$ actuel) d'une personne avec transfert par l'au moins une interface de l'unité de calcul (1) du taux actuel de glucose dans le sang (X$_{BG}$ actuel) à l'unité de calcul (1), l'unité de calcul étant conçue pour calculer la quantité de bolus (Rec) en fonction du taux actuel de glucose dans le sang (X$_{BG}$ actuel) .

3. Dispositif selon la revendication 1 ou 2, comportant un chronomètre (4) qui est couplé à l'unité de calcul (1) afin de transférer l'heure l'unité de calcul (1).

4. Dispositif selon une des revendications 1 à 3, comportant une mémoire (5) qui est couplé à l'unité de calcul (1) et/ou à un chronomètre (4) afin de sauvegarder des paramètres prédéfinis pour le calcul de la quantité de bolus et/ou la quantité d'insuline délivrée et/ou l'évolution temporelle du taux basal (BR) .

5. Dispositif selon une des revendications 1 à 4, comportant un dispositif d'affichage (6) connecté à l'unité de délivrance afin d'afficher la quantité de bolus (Rec) calculée par l'unité de calcul (1).

6. Dispositif selon une des revendications 1 à 5, comportant une pompe à insuline (7) qui est couplée à l'unité de calcul (1) afin d'échanger des informations concernant la quantité de bolus (Rec) et/ou le taux basal momentané (BR$_{actuel}$) .

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6691043 B2
- DE 10057215 A1
- US 20030055570 A

**In der Beschreibung aufgeführte Nicht-Patentliteratur**

- PROSPEKTIVE EVALUATION EINER STANDARD-ISIERTEN BA-SALRATENVERTEILUNG DIE CSII BEI TYP-I-DIABETES ÜBER 6 MONATE. **WIZE-MANN, E. ; RENNER, R. ; HEPP, K. D.** DIABETES UND STOFFWECHSEL, ZEITSCHRIFT FÜR AN-GEWANDTE DIA-BETOLOGIE. DEUTSCHEN DIA-BETES-GESELLSCHAFT, 23. Mai 2001, vol. 10